(19)

Europäisches
Patentamt
European
Patent Office
Office européen
des brevets

(11) **EP 3 198 279 B1**

(12) **EUROPEAN PATENT SPECIFICATION**

(45) Date of publication and mention
of the grant of the patent:
**09.09.2020  Bulletin 2020/37**

(21) Application number: **15775481.3**

(22) Date of filing: **24.09.2015**

(51) Int Cl.:
**G01N 33/68** (2006.01)

(86) International application number:
**PCT/GB2015/052770**

(87) International publication number:
**WO 2016/046558 (31.03.2016 Gazette 2016/13)**

(54) **METHOD OF PROVIDING A PROGNOSIS OF SUCCESSFUL IMPLANTATION OF A CULTURED EMBRYO**

VERFAHREN ZUR BEREITSTELLUNG EINER PROGNOSE FÜR EINE ERFOLGREICHE IMPLANTATION EINES KULTIVIERTEN EMBRYOS

MÉTHODE POUR FOURNIR UN PRONOSTIC D'IMPLANTATION RÉUSSIE D'UN EMBRYON EN CULTURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **24.09.2014  GB 201416866**
**11.06.2015  GB 201510216**

(43) Date of publication of application:
**02.08.2017  Bulletin 2017/31**

(73) Proprietor: **MAP IP HOLDING LIMITED**
**London W3 6JT (GB)**

(72) Inventors:
• **ILES, Raymond Kruse**
**Ely**
**Cambridgeshire CB6 3FQ (GB)**

• **BUTLER, Stephen Andrew**
**Ely**
**Cambridgeshire CB6 3FQ (GB)**

(74) Representative: **Dehns**
**St. Bride's House**
**10 Salisbury Square**
**London EC4Y 8JD (GB)**

(56) References cited:
**EP-A1- 2 975 402        WO-A1-2009/032288**
**WO-A1-2011/075842     WO-A1-2012/066057**
**WO-A1-2015/173107     US-A1- 2010 099 135**

• **NATASA KAHRIC-JANICIC ET AL: "Tandem Mass Spectrometry Improves the Accuracy of Free Thyroxine Measurements During Pregnancy", THYROID., vol. 17, no. 4, 1 April 2007 (2007-04-01), pages 303-311, XP055236310, US ISSN: 1050-7256, DOI: 10.1089/thy.2006.0303**

## Description

The invention relates to a method of providing a prognosis of successful implantation of a cultured embryo comprising subjecting a sample to direct mass spectral analysis, wherein the spectra obtained from the direct mass spectral analysis is normalised; and comparing the normalised spectra obtained with the spectra obtained from a sample of a successfully implanted embryo; wherein the mass spectral analysis is carried out using Matrix Assisted Laser Desorption Ionization Time of Flight mass spectrometry ("MALDI-ToF MS") and the sample consists of culture fluid surrounding the embryo in culture and excludes the embryo itself; and wherein the spectra obtained from the mass spectral analysis is examined for the presence of abnormal peaks in the ranges 22,000-26,000 m/z and 35,000-40,000 m/z indicative of an embryo which is less likely to implant successfully.

## Background

Maternal fetal wellbeing (obstetric care) is central to many world National healthcare services. The increased medicalization of pregnancy from assisted reproduction to emergency delivery due to complications of the pregnancy, such as eclampsia or fetal abnormality/distress, means that a significant proportion of any such budget is spent on Maternity and Reproductive Health. For example in the UK 3.2% of the National Health Service Budget (£3.44 billion in 2010/11) is currently spent on assisted reproduction and obstetric care.

From an emotional and financial perspective (a complicated pregnancy and delivery costs an individual household 10 to 100 times more than that of an uneventful pregnancy), testing to see if the baby is "normal", progressing and "unharmed" is now an expectation of a healthcare service. The rise in GDP expenditure in healthcare over the last 50 years has been due in part to increased testing including that now associate with maternity. Alongside increased accuracy of such tests, there has been exponential rise in costs which have largely been ignored. There is now a need to develop pregnancy screening tests that have high efficacy but affordability to end users be they national health services, health insurance companies or individuals.

Gestational Trophoblastic Diseases (GTD) covers a wide range of pregnancy-related disorders that are in many ways cancerous in nature but can also be considered forms of pregnancy with a spectrum of additional clinical features, morphologic characteristics and sometimes pathologies. GTDs were first described around 400 BC by Hippocrates but finally linked to pregnancy in 1895. GTDs present themselves as premalignant disorders of complete and partial hydatidiform mole or malignant cases such as invasive mole, choriocarcinoma, and the rare placental-site trophoblastic tumour (PSTT). Malignant GTD can be referred to as gestational trophoblastic tumours or neoplasia (GTT or GTN). The initial manifestations of GTD include, vaginal bleeding and vomiting. However, women with gestational trophoblastic tumours present with heavier abnormal vaginal bleeding, and amenorrhea, specifically in placental-site trophoblastic tumour.

As in normal pregnancy hCG tests form a major part of the diagnosis and monitoring of GTD. For many obstetricians a positive pregnancy test in the absence of a viable pregnancy (as confirmed by ultrasound) is immediately indicative of a potential GTD. In some cases, in particular choriocarcinoma, the tumour can be highly invasive and a rapid intervention by an oncologist is essential to preserve the chances of survival for the patient.

However, the management of GTD with hCG tests and ultrasound alone is insufficient to provide the full picture of varied and complicated disorders within the spectrum of GTD. Problems have already been identified in hCG assays which do not discriminate (or discriminate to much) the forms of hCG which can be detected and that in some cases false positives and false negatives have led to misdiagnoses and needless therapies. These issues have led to a better defining of hCG assays in terms of the epitopes recognized in each assay, but it has not circumvented the root problem underlying the metabolic differences resulting in the production of hCG variants. It has been suggested that the hCG form is of

more significance than the absolute amount of total hCG. This qualitative problem needs a qualitative solution.

Hyperemesis gravidarum is defined as persistent vomiting in pregnancy resulting in weight loss, (>5% body mass in the USA, as defined by the ACOG, or >15% body mass in the UK, defined by the RCOG), combined with ketosis, hypokalemia and dehydration. Therefore, it is not simply an exaggerated form of the classic 'morning sickness' which accompanies early pregnancy. Hypovolemic and metabolic derangement, especially that of thyroid function, are potentially life threatening to both the mother and developing fetus. In contrast, the experience of mild and self-limiting nausea and vomiting (morning sickness) are common occurrences in up to 80% of all women during pregnancy.

Early diagnosis of Hyperemesis Gravidarum is clinically important due to the potential harm, as if left unmanaged these symptoms may worsen significantly, leading to loss of the fetus and in some cases result in maternal fatality. Mortality rates from hyperemesis were considerable and as high as 159 deaths per million births, before the introduction of intravenous rehydration therapies. In the United States, the incidence of hyperemesis is much higher than in the UK, and represents an annual hospital admission of 38,000 women.

There is no universally accepted explanation or cure for hyperemesis, with management based on symptomatic approaches, including therapies such as intravenous fluid and electrolyte replacement, thiamine supplementation and psychological support. Nevertheless, early detection and intervention of the disorder is believed to improve management and a biochemical marker in this regard would be useful.

Although no direct link has yet been established, it has often been suggested that hCG could be the most likely cause of hyperemesis gravidarum during pregnancy. This is especially true in cases of women at risk of gestational trophoblastic diseases where hCG levels tend to be unexpectedly high. Older studies revealed that in 36% of the hyperemesis cases examined hCG levels were above the 97.5 percentile of the normal value; however, simply having high hCG is not always consistent with hyperemesis.

Biochemical hyperthyroidism, associated with hyperemesis gravidarum, has been attributed to a mechanism involving hCG cross-reacting agonistically with the thyroid stimulating hormone (TSH) receptors and is identified as a risk factor of hyperthyroidism. Since, the alpha subunits of both TSH and hCG are identical they possess some agonistic effect especially at high circulating concentrations. These thyrotrophic effects of hCG are known to manifest as severe vomiting and nausea, which is the chief symptom of hyperemesis gravidarum; so much so that historically thyroid function tests were a determinant factor in pregnancy diagnosis and patient management. Furthermore, acidic isoforms of hCG have been speculated to be a causative factor in protracted cases of severe vomiting and nausea. The underlying explanation is that that acidic forms of hCG, due to the presence of additional sialic acid rich glycosylations, promotes a longer circulating half-life of the hormone, and thus an exacerbation of the thyrotrophic effects described. Significantly recent studies indicated that the involvement of hCG in hyperemesis might be structural rather than simply quantitative and a thyrotropic form of hCG has been suggested.

**Pre-eclampsia**

Pre-eclampsia is a disorder of pregnancy characterized by high blood pressure and large amounts of protein in the urine. This can lead to impaired liver function, kidney dysfunction, pulmonary oedema, and neurological and visual disturbances. If left untreated, preeclampsia can develop life-threatening occurrence of seizures during pregnancy, termed eclampsia. Even if detected early pre-eclampsia is associated with multiple maternal and fetal adverse effects. Affecting between 2-8% of pregnancies worldwide, it may develop from as early as 20 weeks of gestation, though most commonly after 32 weeks. Pre-eclampsia occurring before 32 weeks is considered early-onset and is associated with increased morbidity. Most cases are diagnosed before term and delivery of the fetus and placenta is the only known treatment for pre-eclampsia.

The pathogenesis of preeclampsia has not been determined although abnormal placentation is a strong predisposing factor. There are a host of contributing and related factors that include maternal immunological responses. Central to the effects of preeclampsia are the resulting presence of utero-placental hypoxia, an imbalance in angiogenic and anti-angiogenic proteins, oxidative stress, maternal vascular endothelial dysfunction, and elevated systemic inflammation. Given the multi-factorial nature of the disease, it is not yet possible to routinely predict preeclampsia.

**Multiple pregnancy.**

A multiple pregnancy occurs when the women is carrying more than one embryo or foetus, such a twins, triplets, quadruplets etc. Detecting a multiple pregnancy is usually established in the weeks 10-13 of pregnancy following demonstration of multiple embryonic sacs by ultrasound. The analysis of protein markers to indicate pregnancy disorders have been used for decades but proteomic analysis of the serum and urine are relatively new approaches especially to identify twins.

**Spontaneous abortion or miscarriage**

Spontaneous abortion (also known as miscarriage) is a common complication of early pregnancy and occurs when the embryo or foetus dies naturally within the first 20 weeks of pregnancy. Among women who know they are pregnant, the miscarriage rate is approximately 10% to 20% while rates among all conceptions is around 30% to 50%. About 5% of women have two miscarriages in a row, but women can have multiple miscarriages. Knowing about this early could offer obstetricians an opportunity to intervene and potentially reduce the risk of losing the pregnancy.

**Embryo / Early Pregnancy**

Human chorionic gonadotrophin (hCG) is one of the most widely studied markers in embryonic development. Despite its worldwide usage as an obstetric marker, hCG is often regarded as little more than a signal for maternal recognition of pregnancy through the subsequent maintenance of progesterone production. The hCG molecule has several isoforms attributed to varying degrees of glycosylation and stages of metabolic degradation, some of which may now have more specific functions different to that of "normal" hCG. The normal or regular or intact hCG (as it is often referred, and subsequently indicated as hCG in this example) is the predominant form of hCG produced by the trophoblast during pregnancy, and is a heterodimer consisting of a hCG$\alpha$, and hCG$\beta$ subunit; it is also often erroneously referred to as beta hCG and for the purpose of this example needs clarification, the beta subunit of hCG is distinct.

Hyperglycosylated hCG (hCGh or invasive trophoblastic antigen, ITA) is a form of hCG produced by during pregnancy especially during the first trimester. hCG$\beta$ is the free form of the hCG$\beta$ subunit arising either through dissociation of hCG or through ectopic expression in the absence of hCG$\alpha$. There have been numerous studies on the expression and production of hCG by developing embryos in culture, however few have linked the production of specific hCG isomers to particular function or dysfunction, and only more recently suggested that variability of hCG concentrations *in vitro* may act as a marker for implantation potential. Several studies have suggested that measuring hCGh on its own can provide an accurate prediction of pregnancy failures. Others reported that hCGh can be used to identify a term pregnancy against failing pregnancy on the day of implantation with high accuracy. Thus, it can be useful to identify this hCG isoform in order to differentiate a successful pregnancy from that of a potential failure. It has also been noted that a correlation exists between early embryo proteomic, secretomic, and metabolic profiling, and successful implantation rates. Therefore, hCG isoform profiling in the secretome may be the key for identification of viable embryos, particularly in a non-invasive manner. This is important, considering that despite recent studies suggesting several metabolic and biochemical markers for predicting implantation, none have yet been found of any clinical value.

WO2009/032288 discloses methods for predicting the viability of an embryo or for predicting the likelihood of a negative outcome during pregnancy by identifying the presence or absence of or determining the amount of one or more pregnancy associated markers such as molecular isoforms of hCG in a sample, as well as methods for determining the amount of a pregnancy associated markers such as molecular isoforms of hCG (hCG) in a sample; and a diagnostic kit for predicting the viability of an embryo or for predicting the likelihood of a negative outcome during pregnancy by identifying the presence of or determining the amount of one or more pregnancy associated markers such as molecular isoforms of hCG in a sample.

US2010/0099135 discloses methods and assays for assessing the presence, viability and/or developmental stage of an embryo, as well as method and assays for assessing the quality of an Assisted Reproduction Technology protocol.

Natasa Kahric-Janicic et al., THYROID 17(4): 303-311 discloses a study, the objective of which was to characterize the performance of a tandem mass spectrometric assay for free thyroxine during the physiologic conditions of pregnancy. Design: Healthy women without a history of thyroid abnormalities were recruited from the obstetrics and gynecology and endocrinology clinics of a university medical center and their thyroid status was monitored. Free thyroxine levels were assessed by both immunoassay and tandem mass spectrometry during the course of their pregnancy. Serum thyrotropin levels were also measured. The distributions of free thyroid concentrations obtained by the two assays were compared. Main outcome: The tandem mass spectrometry and immunoassay values did not correlate well with each other. However, tandem mass spectrometry values correlated well with the current gold standard equilibrium dialysis values. Moreover, the good agreement between equilibrium dialysis and tandem mass spectrometry was maintained across all weeks of gestation.

WO2011/075842 discloses a panel of metabolites that are associated with early detection of predisposition to pre-eclampsia. In some embodiments, the risk may be detected early in pregnancy, for example at 15 weeks gestation, by measuring the level of selected compounds in maternal serum. The compound may be one or more of 5-hydroxytryptophan, a monosaccharide, decanoylcarnitine, methylglutaric acid, adipic acid, oleic acid, docosahexaenoic acid, docosatriynoic acid, y-butyrolactone, oxolan-3-one, 2-oxovaleric acid, oxo-methylbutanoic acid, acetoacetic acid, hexadecenoyl-eicosatetraenoyl-sn-glycerol, Di-(octadecadienoyl)-sn-glycerol, sphingosine 1- phosphate, sphinganine 1-phosphate or a vitamin D3 meabolite.

WO2012/066057 discloses a method of predicting a SGA infant in a patient at a pre-symptomatic gestational stage. The method comprises a step of assaying a biological sample from the patient for an abundance of a plurality of metabolite biomarkers selected from 19 metabolite biomarkers, correlating the abundance of the plurality of metabolite biomarkers with a metabolite fingerprint of SGA, and predicting SGA based on the level of correlation between the abundance of the plurality of metabolite biomarkers and the metabolite fingerprint.

EP2975402, which is prior art under Article 54(3) EPC, discloses a non-invasive method of measuring haptoglobin fragments in human serum albumin supplemented culture medium of *in vitro* cultured embryos as part of an ART procedure, which serves as an alternative or supportive method to assess the viability of *in vitro* cultured embryos without doing any harm to the embryos.

WO2015/173107 discloses a non-invasive method for the early diagnosis of fetal malformations based on the metabolomics analysis of maternal blood. The subject-matter of WO2015/173107 is prior art under Article 54(3) EPC, with the exception that the subject-matter of WO2015/173107 which is not entitled to the claimed priority date of 15 May 2014 is not prior art against the presently claimed subject-matter entitled to the claimed priority date of 24 September 2014.

US2009/0075293 describes a method for predicting embryo viability by identifying the presence or absence of one or more pregnancy associated markers such as molecular isoforms of hCG in a sample. High quality embryos were distinguished from low quality embryos based on the embryo's ability to utilize/metabolise proteins in the molecular weight range 9149-9157, thought to be an isoform of the CRAb protein. The samples were processed prior to analysis, by treatment with DTT and the removal of albumin. The method is based on identifying the presence or absence of certain proteins. The method of the present invention does not require the identification of specific proteins. The present invention looks for changes in the overall profile of the spectra, or in certain regions as compared to the spectra obtained from an equivalent sample from healthy ongoing pregnancies.

The method herein describes analysis of culture fluid that surrounds an embryo in culture, excluding the embryo itself, during assisted reproductive technology procedures by direct mass spectral analysis, wherein the mass spectral analysis is carried out using Matrix Assisted Laser Desorption Ionization Time of Flight mass spectrometry ("MALDI-ToF MS"). Analysis may be carried out directly on a sample or following dilution, for example in distilled deionised water, or other suitable diluent. Optionally dilution at the range of 1/2 to 1/1000 in for example distilled deionised $H_2O$ or 0.1% trifluoroacetic acid (TFA) in distilled deionised $H_2O$ may be necessary for concentrated samples. The resulting spectra are normalised and the normalised spectra obtained are compared with the spectra obtained from a sample of a successfully implanted embryo, wherein the spectra obtained from the mass spectral analysis is examined for the presence of abnormal peaks in the ranges 22,000-26,000 m/z and 35,000-40,000 m/z indicative of an embryo which is less likely to implant successfully. This provides a prognosis for pregnancy. It is not necessary to look for any certain known proteins, or to identify the proteins which cause the change in profile.

The present invention provides a method of providing a prognosis of successful implantation of a cultured embryo as defined in the appended claims.

"Direct mass spectral analysis" means that the data generated from the mass spectral analysis is used in the method, and not the inferred mass of the components present in the sample.

The sample is a sample consisting of the culture fluid surrounding an embryo in culture and excludes the embryo itself.

"Embryo" as used here in refers to any cell or group of cells post fertilization, but prior to intrauterine transfer. It includes zygotes, blastomeres, morulae, and blastocysts.

"Prognosis for a pregnancy" as used herein refers to predicting the likelihood of an embryo implanting successfully, as well as, diagnosing or predicting the likelihood, i.e. providing a prognosis of a pregnant woman suffering from a disorder of pregnancy, or having a multiple pregnancy. Disclosed is a method used to detect embryos with chromosomal abnormalities. A prognosis for pregnancy can be a successful ongoing pregnancy or a failing pregnancy. As used herein an "ongoing" pregnancy is a normal pregnancy that is expected to proceed to term and result in a live birth. A "failing pregnancy" as used herein refers to a biochemical pregnancy, or a pregnancy resulting in a spontaneous abortion/miscarriage, recurrent miscarriage.

Disclosed is a method used to provide a prognosis and/or diagnosis of a disorder of pregnancy, as well as a prognosis for a multiple pregnancy. For these disclosed methods the sample is obtained from a pregnant woman. As used herein a "disorder of pregnancy" includes Ectopic pregnancy, biochemical pregnancy, early pregnancy loss (EPL), Threatened Miscarriage, Spontaneous abortion or miscarriage, Hyperemesis Gravidarum and Gestational Trophoblastic Diseases, Placental Insufficiency, Pre-eclampsia, Gestational Diabetes, Placenta previa, placenta accreta, placenta percreta, Obstetric Cholestasis, and Recurrent Miscarriage in both normal and assisted reproduction. Gestational Trophoblastic Diseases include molar pregnancies, choriocarcinoma, and placental trophoblastic tumour (PSTT). The term "disorder of pregnancy "as used herein, when referring to a sample obtained from a woman (i.e. not an embryo culture fluid sample) excludes conditions within the fetus such as fetal aneuploidy.

Disclosed is a method of screening for pregnancy disorders which are already present when the sample is obtained such as Ectopic pregnancy, Threatened Miscarriage, Hyperemesis Gravidarum and Gestational Trophoblastic Diseases, as well as multiple pregnancy. Disclosed is a method that can provide an indication of the risk of developing other disorders of pregnancy which generally occur later in the pregnancy (i.e. after the sample has been taken) such as Placental Insufficiency, Pre-eclampsia, Gestational Diabetes, Obstetric Cholestasis, Recurrent Miscarriage, and spontaneous abortion in both normal and assisted reproduction. Thus the disclosed method has both diagnostic and prognostic value. Preferably the disorder of pregnancy is selected from Pre-eclampsia, Hyperemesis Gravidarum, or Gestational Trophoblastic Diseases. In one disclosure the disorder of pregnancy is not a Gestational Trophoblastic Disease. Preferably the disclosed method is used to provide a prognosis of a multiple pregnancy or a spontaneous abortion.

A "multiple pregnancy" as used herein refers to a pregnancy where the woman is carrying more than one embryo or foetus, such as twins, triples, quadruplets, quintuplets, etc.

A "biochemical" pregnancy as used herein refers to the situations where a pregnancy test provides a positive result, but the embryo fails to implant properly, so does not result in an ongoing pregnancy.

The disclosed sample obtained from a pregnant woman is a bodily fluid sample. Bodily fluids include cerebrospinal fluid, seminal fluids, vaginal fluids, interstitial fluids, tissue aspirates, saliva, and urine such as early morning and midstream urine samples, blood and serum. The sample may be a spot card sample, for example a urine or blood spot card sample, wherein the sample is applied to filter paper or other capture material, allowed to dry and stored for future analysis. The blood sample can be a whole blood sample collected using conventional phlebotomy methods. For example, the sample can be obtained through venupuncture or as a pin prick sample, such as a finger-stick or heel prick. The blood sample may be a dried blood spot captured on filter paper or other suitable blood spot capture material. Preferably the sample is a urine sample.

The disclosed sample from a woman may be obtained at any point during the pregnancy. In one preferred embodiment the sample may be obtained from a subject in the first trimester of the pregnancy, for example 4-13 weeks gestation, preferably 6-11 weeks gestation, 5-7 weeks gestation and/or 8-11 weeks gestation. The sample may also be obtained during the second trimester or later, i.e. 14 or more weeks gestation, preferably 16-35 weeks, more preferably 18-30 weeks gestation.

The method of the invention can be used to give a prognosis of successful implantation of a cultured embryo, provided by assisted reproduction. The method can give a prognosis for a successful implantation followed by an ongoing pregnancy or a failing pregnancy such as a spontaneous abortion (miscarriage), a biochemically positive pregnancy, as well as detect chromosomal abnormalities in the embryo. The method is carried out on a sample of the fluid (such as buffered saline, embryo or blastocyst culture media) that surrounds an embryo in culture during assisted reproductive technology procedures. This includes the fluid in which the embryo is stored or frozen. It also includes any fluid arising in the culture media from the embryo itself, for example after blastocyst hatching. The sample can be taken at any time prior to transfer. Preferably the sample is taken 3-5 days post-fertilization. The sample can be taken either prior to, or after blastocyst formation. The sample can be taken shortly i.e. 0.5-12 hours, before transfer to the recipient.

"Chromosomal abnormalities" as used herein refer to any condition where the cell has a variation from the normal chromosome number e.g. 46 in humans. These include fetal aneuploidies, such as Down's Syndrome, Patau syndrome Turner Syndrome, Klinefelter syndrome, Edwards syndrome and triple-X as well as fatal trisomies of any other of the 23 chromosomes.

The sample (embryo culture fluid) may be a neat sample. Alternatively, the sample may be diluted or processed (concentrated, filtered, etc.).

Preferably the sample (embryo culture fluid) is diluted. The sample may be diluted 1/2 (i.e. one part sample in 1 part diluent), 1/5, 1/10, 1/100, 1/200, 1/500, 1/1000, 1/2500 or more. Most preferably the sample is diluted 1/1000 i.e. one part sample in 1000 parts diluent. Preferably the diluent is water or 0.1% trifluoroacetic acid (TFA) in distilled deionised $H_2O$, more preferably distilled deionized water.

If the sample is stored on a spot card or blood spot capture material, it can be reconstituted using a suitable buffer, or a diluent. Preferably the diluent is water or 0.1% trifluoroacetic acid (TFA) in distilled deionised $H_2O$, more preferably distilled deionized water.

Preferably the sample (embryo culture fluid) is not processed prior to dilution. Such processing includes concentrating the proteins of interest e.g. hCG; isolating hCG by for example HPLC, removal of contaminating proteins e.g. albumin, protease e.g. trypsin digestion or treatment with a chemical agent to disrupt or break intramolecular bonds. In particular, the sample is preferably not treated with a reducing agent. More preferably the sample is not treated with dithiothrietol (DTT). The sample is preferably not treated with DTT if it is a urine sample.

During direct mass spectral analysis, a spectra is generated using a matrix. Suitable matrix compounds include sinapinic acid, ferulic acid (FA) and alpha 4- cyano hydroxycinnamic acid (CHCA). The intensity of the characteristic resolved mass peaks are measured as specific m/z values ranges or a ratio determines the relative abundance of specific m/z peaks. The spectra obtained from the mass spectral analysis is examined for the presence of abnormal peaks in the ranges 22,000-26,000 m/z and 35,000-40,000 m/z indicative of an embryo which is less likely to implant successfully. Further, the spectra may be analyzed over the range of 1000 m/z to 100,000 m/z. Preferred ranges include 2000 m/z to 100,000 m/z, 4,000 m/z -35,000 m/z, 4,000 m/z - 80,000 m/z, 10,000 m/z - 75,000 m/z, 70,000 m/z - 80,000 m/z 2,000 m/z - 14,000 m/z; 6,000 m/z - 14,000 m/z, 6,000 m/z - 8,000 m/z, 12,000 m/z - 50,000 m/z 20,000 m/z - 50,000 m/z; 22,000 m/z - 26,000 m/z; 30,000 m/z - 40,000 m/z; and 35,000 m/z - 40,000 m/z, or combinations thereof.

The spectra as a whole can be analysed, or one or more subranges, or regions can be analysed e.g. 2,000 m/z to 16,000 m/z, and 70,000 m/z - 80,000 m/z Thompson Units (Th) identify a region of mass: charge (m/z) which is itself proportional to mass in kDa.

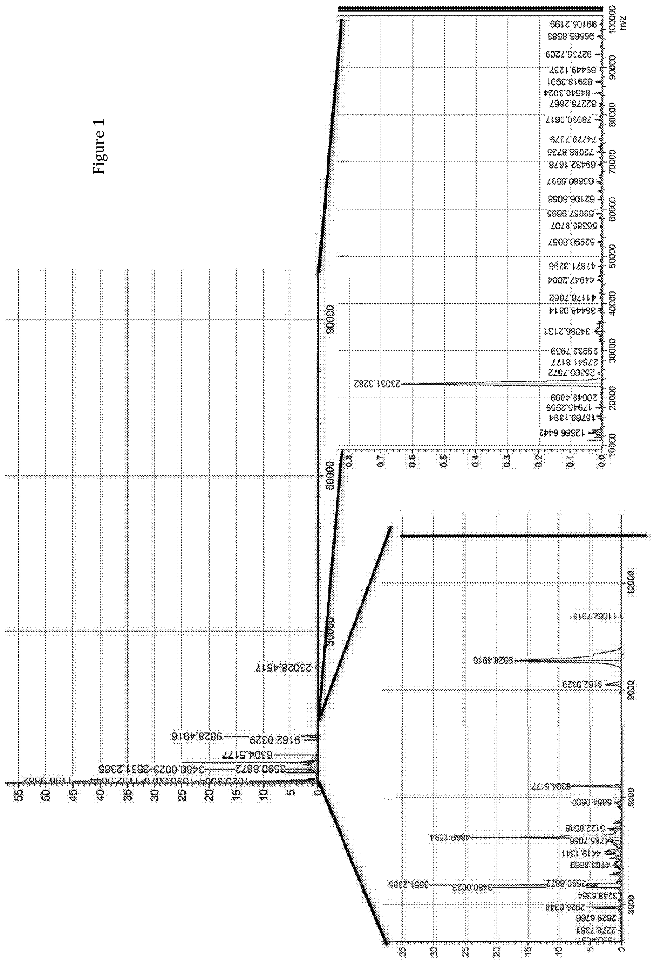

Clusters of peaks are usually seen in urine, as shown in Figure 1, at:
2,000 m/z to 6,000 m/z - predominately, but not exclusively, arising from metabolites of trypsin inhibitors.

6,000 m/z to 14,000m/z - predominately, but not exclusively, arising from the beta core metabolite of human chorionic gonadotrophin.

14,000 m/z to 100,000m/z predominately, but not exclusively, molecular variants of hCG and its alpha and beta subunits.

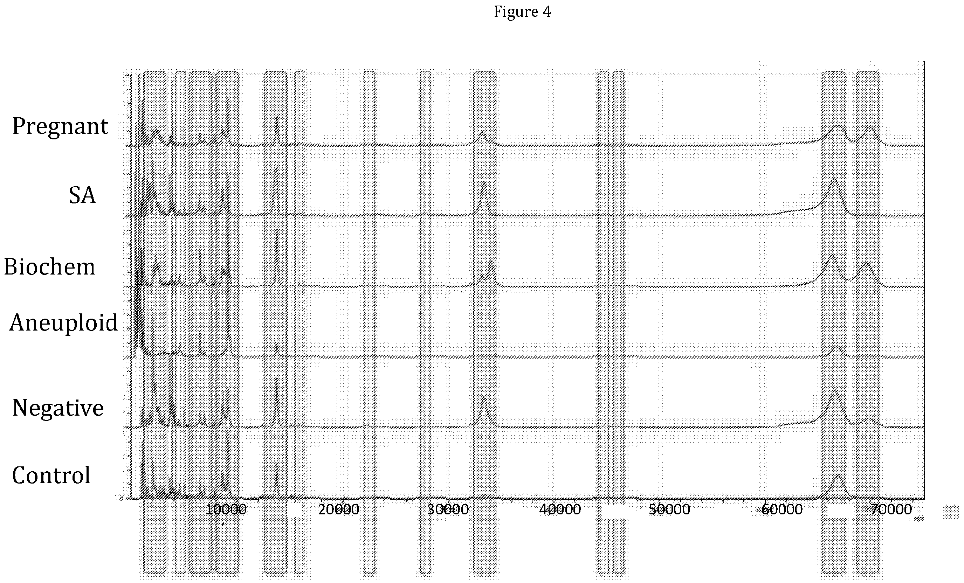

Peaks usually seen in embryo culture fluid vary between different pregnancy outcomes as shown in Figure 4 (and 9).

Methods of generating mass spectra, such as mass spectrometry including MALDI-Tof MS, are commonly not quantitative techniques. For example the Y axis in these spectra is an indicator of "relative strength" of mass peak within the spectra, but not between mass peaks in one sample versus another sample. In order to overcome this, normalization needs to render Y axis value comparable between sample spectra. Thus the spectra obtained from the direct mass spectral analysis are normalized. The spectra are subjected to data processing which results in a normalized statistically determined index of relative proportion of mass spectra. This converts the qualitative mass spectra into a quantitative value. Normalization is the process of producing a data structure to reduce repetition and inconsistencies of data. Several normalization techniques are possible. Typical normalization methods include percentage of total area at a given point, Square difference and ratio of differences. The percentage difference is calculated as

$$\text{Percentage difference} = (Yl\text{-}Yref/\ Y\ ref\ X\ 100\%)$$

Wherein Y ref is the minimum measured Y value of the spectra, and Yl is Y value for each point.
The square difference is calculated as

$$\text{Square Difference} = (Yl - Y\ ref)^2$$

The ratio difference is calculated as

$$\text{Ratio Difference} = (Ratio\ 1\text{-}Ratio\ 2).$$

Thus the data from the mass spectra is manipulated (normalized) in order to provide a quantitative measure of the qualitative change shown on the spectra.
Preferably, the spectral model is created by a method of data processing which results in a normalized statistically determined index of relative proportion of mass spectra within a set range. This renders all spectra comparable such that the median and centile variability at any given mass value can be modeled.
A normalized statistically determined index of relative proportion of mass spectra within a given range can be calculated from using the total area under the curve of the mass spectra in a defined area of interest or combinations thereof e.g. 2000-14000m/z; 6000-14,000m/z; 12,000 m/z - 50,000 m/z, 30,000 m/z - 40,000 m/z and 14,000 to 100,000/m/z. This can then be used to calculate the relative intensity of mass regions that alter in samples from patients with particular disorders.
The area under the curve of mass spectra is calculated by dividing the mass spectra into a plurality of bins of a given number of m/z. As used herein "Bin" has its usual statistical meaning, for example, of being one of a series of ranges of numerical value into which data are sorted in statistical analysis. For example the bins can be 100m/z, 50m/z, 25m/z, 10m/z, 5m/z or 3m/z in size. The smaller the size of the bin used, the more refined the method. If two or more regions of interest within the spectra are analysed, then different sized bins can be used for reach region. For example the range 1,000-10,000m/z can be analysed using 5m/z bins together with the range 14,000-100,000m/z analysed using 10m/z bins.
The relative intensity (Y Axis value) can be calculated by the "square of difference" method and therefore a comparable Y value given for every bin. In this method, the minimum Y value of the spectra (Y ref) was subtracted from the Y value at every bin and the difference was squared. The formula used to calculate square of difference = $(y1\text{-}yref)^2$ and the calculated square of difference was then named as "relative intensity".
The relative intensity at each mass bin in a sample can be captured using commercially available statistical tests such as MATLAB ®, Stats Direct™ and Origin 8™.
Each sample is compared against a reference spectral model. The "reference spectral model" is the expected mass within a set range, determined from statistical analysis of a collection of samples obtained for normal healthy pregnancies. As used herein a "normal healthy" pregnant woman is one who has an ongoing pregnancy following successful implantation of a cultured embryo. Preferably the reference spectral model of expected mass is determined from statistical analysis of a collection of samples at matched gestational age. The sample is compared to a reference spectra generated from the same sample type e.g. a culture fluid sample is compared to a reference spectra generated from embryo culture fluid, and not a maternal sample. Any changes, such as a change in mass, an increase or decrease in the relative intensity, or a change in the ratio of relative intensity between two or more peaks in the sample spectra as compared to the reference spectra may be indicative of a change in the prognosis of successful implantation. For example, an increase in the relative intensity of a peak associated with a disorder of pregnancy may be indicative of the disorder of pregnancy being present. Preferably the range is between about 500 m/z - 100,000m/z, for example 1,000 m/z - 75,000 m/z, 2,500 m/z - 50,000m/z , 5,000 m/z - 25,000m/z, 6,000 m/z -14,000 m/z or 12,000 m/z - 50,000 m/z or combinations thereof. Most preferably the range is 6,000 m/z -14,000 m/z. Preferably the spectral model of expected mass is determined from statistical analysis of a collection of samples of the same type e.g. embryo culture fluid at matched gestational or embryo age.

Preferably, the spectral model is created by a method of data processing which results in a normalized statistically determined index of relative proportion of mass spectra within a set range. This renders all spectra comparable such that the median and centile variability at any given mass value can be modeled.

Preferably, a parallel "disease" model, as generated above from normalized statistically determined index of relative proportion of mass spectra within a set range is created from culture fluid samples from embryos which failed to successfully implant. The spectra from a sample can then be compared to the disease model generated from samples of the same type. The presence of a change in mass or peak associated with an embryo less likely to implant may be indicative of a poor prognosis for implantation and/or pregnancy.

Once the spectra has undergone a method of data processing which results in a normalised statistically determined index of relative proportion of mass spectra any significant changes in mass can be attributed to a given disorder or diagnostic utility.

The reference spectral model and the disease model, are then compared by plotting in order to identify 'hot spots' i.e. points or regions of difference between the two models (for example as shown in Figure 4). This may be a decrease or increase in the size of a peak, or the appearance of a peak. The points of difference can then be used to determine the prognosis of successful implantation. This may be done by using a suitable algorithm.

Disclosed ranges and combinations thereof for analysis in varous conditions are provided below:
Gestational trophoblastic disease (GTD): 35,000 - 45,000m/z, preferably 37,600-39,600 m/z

Hyperemesis gravidarum: 22,000-40,000 m/z ; preferably 11,000-26,000 m/z, and/or and/or 32,500-34,500 m/z, and/or 35,000-37,000 m/z and/or 37,500-40,000 m/z .

Pre-eclampsia: 2,000-100,000 m/z ; preferably 14,000-100,000m/z and/or 2,000 - 14,000m/z; especially 6,000 - 8,000m/z.

Successful of embryo implantation: 22,000-40,000 m/z ; preferably 22,000-26,000 m/z and/or 35,000-40,000 m/z.

Failing implantation : 9700-10,000 m/z and/or 7,500 - 8,000 m/z and/or 10,500-12,000 m/z.

Outcome of pregnancy from embryo culture fluid: 12,000-50,000m/z. Preferably 4600-4700 m/z and/or 6400-6500 m/z and/or 9100-9200 m/z. Alternatively preferred range include 15,800-16,100 m/z and/or about 36,400 m/z and/or 9,000-9500 m/z, and/or 8,400-8,700 m/z and/or about 28,000 m/z.

Spontaneous abortion : 6000-12000 m/z. Preferred ranges include 6000-6050 m/z and/or 6450-6500 m/z and/or 9350-9400 m/z and/or 11150-11200 m/z and/or 11800-11850 m/z.

Mutiple gestations: 6000-14000, preferably 9000-13000. Other preferred ranges include 12500-12550 m/z and/or 10300-10350 m/z and/or 9600-9650 m/z.

For all of the conditions listed above analysis can be carried out within a single range or a combination of any of the ranges listed.

Disclosed is a method used to detect chromosomal abnormalities such as aneuploidy in an embryo by analyzing the embryo culture fluid. Current methods for detecting chromosomal abnormalities in embryos require removal of a cell from the developing embryo which is then used for genetic analysis. This can be PGS (put in the full name) where simple karyotyping is carried out to identify the number of chromosomes present and thus aneuploidy. Alternatively genetic conditions can be identified by looking for sequence abnormalities. The disclosed method allows chromosomal abnormalities to be identified from the culture fluid, and thus eliminates any risk of harm to the embryo, as it is not necessary to remove a cell.

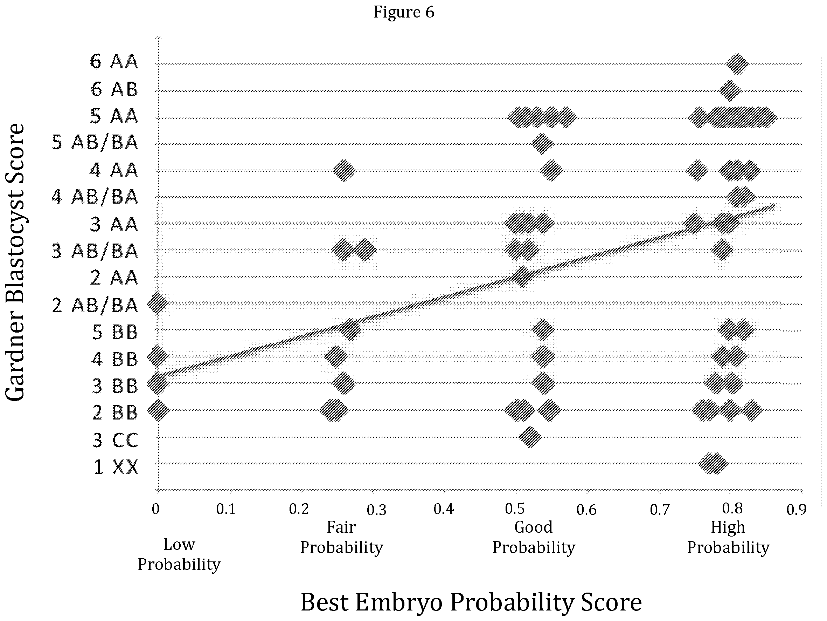

Preferably, the method is used to provide a prognosis for successful embryo implantation, prior to embryo transfer. The likelihood of an embryo successfully implanting after transfer can be assessed by analysing the spectra in the range 22,000 m/z - 40,000 m/z from a sample of culture fluid surrounding the embryo. The presence of abnormal peaks in the ranges about 22,000 m/z to 26,000 m/z and about 35,000 m/z - 40,000 m/z in a culture fluid sample may be indicative of an embryo which is less likely to implant successfully. The sample is preferably obtained at day 3-5 post fertilisation. The results obtained can be used to score the embryo based on the likelihood of successful implantation, resulting in an ongoing pregnancy. The results obtained using the method of the invention are improved compared to the conventional (Gardner scoring) methods (see Fig 6). In addition reliable results can be obtained by the method of the invention at an earlier stage e.g. day 3 post fertilisation as opposed to day 5, immediately prior to transfer. This provides more time for decision making and embryo selection.

Disclosed is a method is used to provide a prognosis for successful embryo implantation, after embryo transfer. Changes in mass within a urine sample from a woman post embryo transfer can also indicate whether an embryo has successfully implanted. Urine samples from women post embryo transfer which have a reduction or elimination of peaks in the range about 9,700 m/z to 10,000 m/z, and/or abnormally elevated peaks around about 7,500 m/z to 8,000m/z and about 10,500 m/z to 12,000 m/z, are indicative of failing implantation .

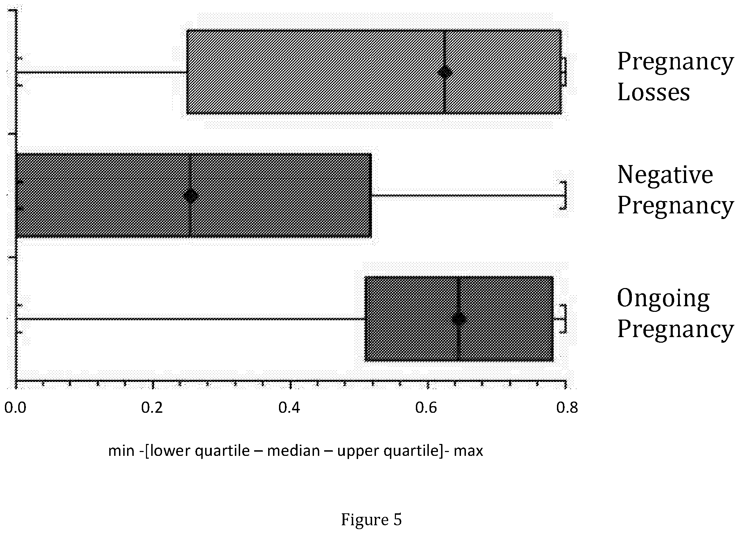

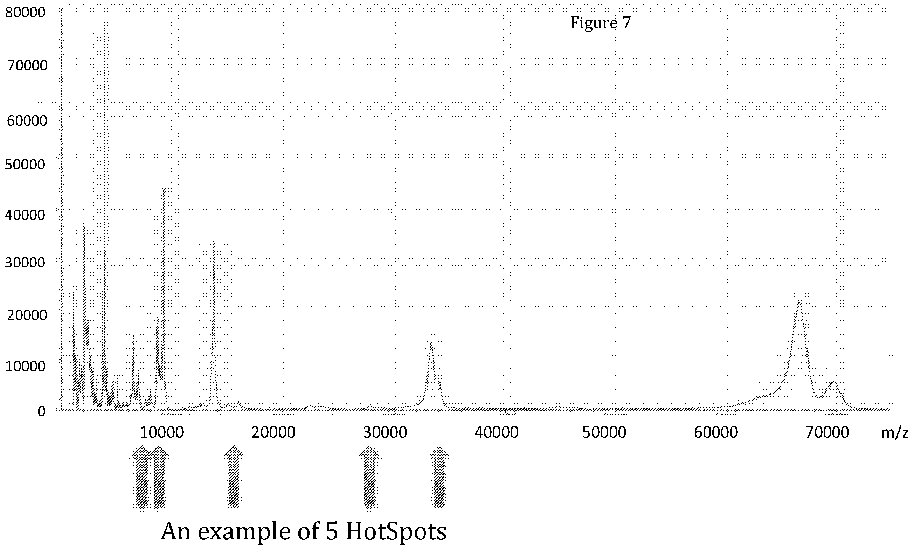

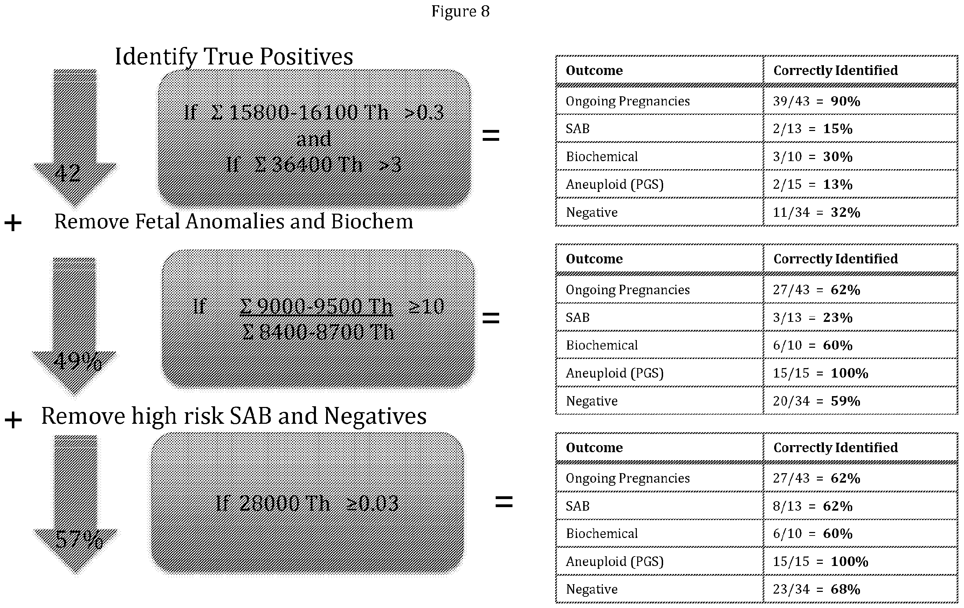

The specific regions from 4600 to 4700 m/z, from 6400 to 6500m/z and from 9100 to 9200 m/z were identified as areas of mass spectral differences between samples, or "hot spots" which can be used to provide a prognosis of different outcomes of pregnancy in the culture fluid surrounding embryos. (See figure 5). In addition areas of difference can be used to create a Predictive Algorithm. As shown in Figures 7 and 8 hot spots can be identified in the spectra, and then these areas of difference can be used to build an algorithm which can be used to provide a prognosis of pregnancy. Peaks in the ranges about 15,800 m/z to 16,100 m/z, about 36,400 m/z, about 9,000 - 9,500 m/z, about 8400 m/z -8700 m/z and about 28,000m/z and combinations thereof in a culture fluid sample can be used in to develop an algorithm. Methods of generating a suitable algorithm are known to the skilled person.

Disclosed is a method used to detect multiple gestations by analysis of a maternal sample. Detecting a twin or triplet pregnancy is usually established early in pregnancy following demonstration of multiple embryonic sacs by ultrasound. The analysis of protein markers to indicate pregnancy disorders have been used for decades but proteomic analysis of the serum and urine are relatively new approaches especially to identify twins. The disclosed method can be used to correlate the spectral analysis of urine by MALDI ToF MS to identify multiple from singleton pregnancies.

The disclosed range for analysis for the detection of multiple gestations is 6,000 m/z - 14,000 m/z. Peaks in the ranges about 10,300 m/z to 10,350 m/z, about 9,600 - 9,650 m/z, and about 12500 m/z -12550 m/z and combinations thereof in a maternal sample can be used in to develop an algorithm or decision tree. Changes in the protein profile as compared to a normal pregnancy occur in both ranges.

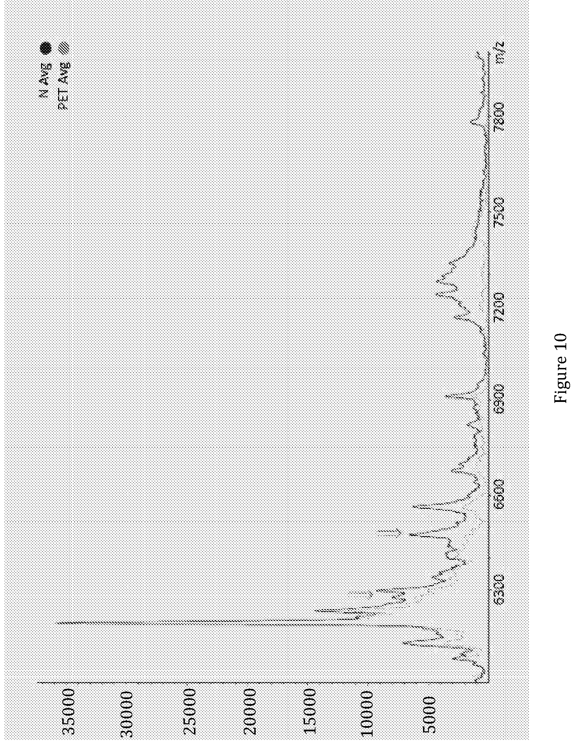

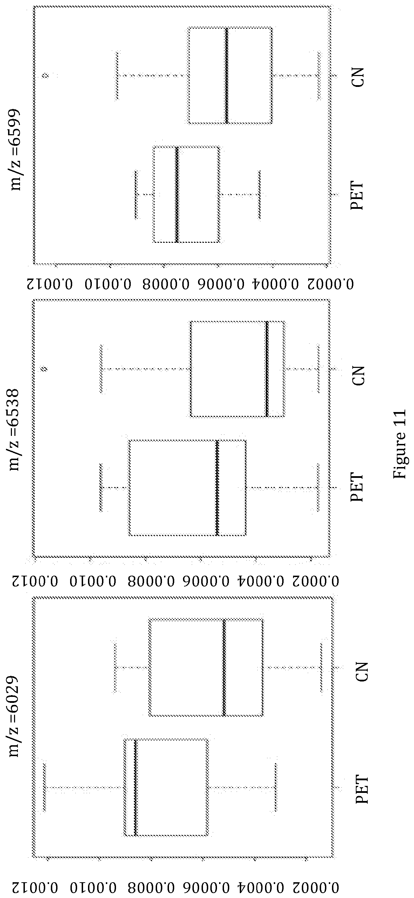

Disclosed is a method used to provide a prognosis for and/or detect pre-eclampsia by analysis of a maternal sample. The disclosed range for analysis for the diagnosis and/or prognosis of pre-eclampsia is 14,000 m/z - 100,000 m/z and also in the low mass range of 2,000 m/z - 14,000 m/z, preferably 6,000- 8,000 m/z. Changes in the protein profile as compared to a normal pregnancy occur in both ranges. In a disclosure, preferably a mass shift in the 2,000 m/z - 14,000 m/z range in a sample taken in the first trimester is indicative that pre-eclampsia is likely to develop at a later stage. Changes in the peaks at 6029m/z, 6538m/z and/or 6599 m/z $\pm$ 5m/z are indicative of pre-eclampsia as shown in Figures 10 and 11. Therefore a positive result is indicative that the patient should be given prophylactic treatment, e.g. aspirin and low molecular weight heparin.

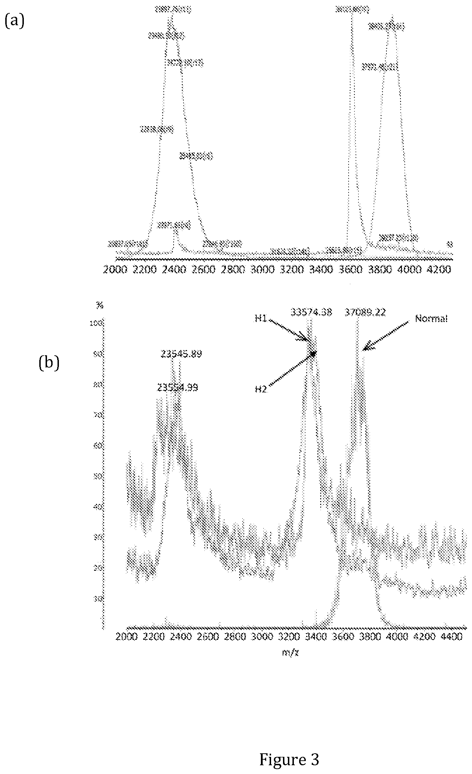

Disclosed is a method used to detect the presence of Hyperemesis gravidarum from analysis of a maternal sample. For Hyperemesis gravidarum the disclosed range for analysis is 22,000 m/z to 40,000 m/z. In samples from women with a normal pregnancy peaks are seen within the range 22,000 m/z to 26,000 m/z, 35,000 m/z to 37,000 m/z and 37,500 m/z to 40,000 m/z. In samples from hyperemesis patients abnormal peaks around 32,500 m/z to 34,500 m/z appear, which are indicative of the condition. The peak seen in normal pregnancy samples at 37,089.22 m/z is shifted to a lower m/z, so that abnormal peaks appear at 33,670.68 for H1 and 33,674.34 for H2 in Hyperemesis gravidarum patients as shown in Figure 3.

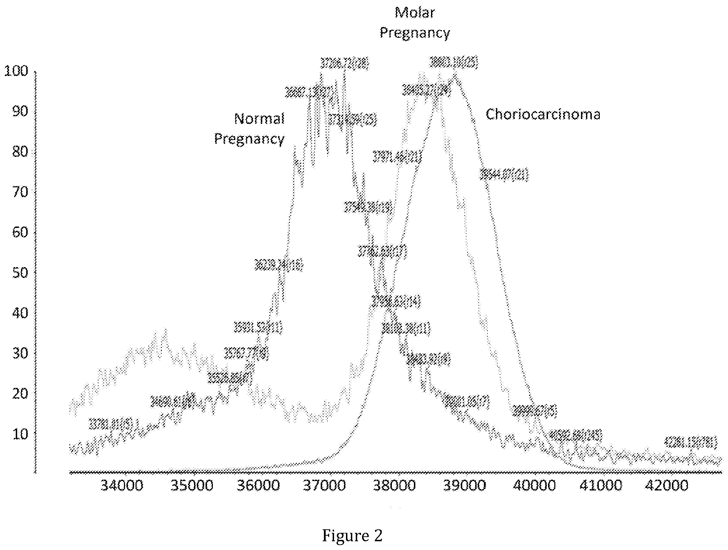

Disclosed is a method used to detect the presence of a gestational trophoblastic disease from analysis of a maternal sample. For gestational trophoblastic diseases (GTD), the disclosed range for analysis is 35,000 m/z to 45,000 m/z. A mass shift of the peaks seen within 35,000 m/z to 37,500 m/z in a sample from patients with normal pregnancy occurs in GTD. A shift of the peaks to the range about 37,600 m/z to 39,200 m/z can be indicative of molar pregnancy and, a shift of the peaks to the range about 37,900 m/z to 39,600 m/z can be indicative of choriocarcinoma. In particular a peak around 38,405 m/z is seen in patients with a molar pregnancy and, a peak around 38,803 m/z is seen in patients with choriocarcinoma, as compared to that seen in normal pregnancy, which is around 36,687 m/z., as shown in Figure 2.

The analysis of the mass spectra can be easily calculated using a suitable computer software program. A computer can also be programmed with the suitable algorithm in order to provide an indication of the likelihood of a pregnancy disorder, or successful implantation.

Direct mass spectral analysis can be carried out by mass spectrometry. Disclosed mass spectrometry techniques include fast atom bombardment (FAB), chemical ionization (CI), atmospheric-pressure chemical ionization (APCI), electrospray ionization (ESI), matrix-assisted laser desorption/ionization (MALDI), Quadrupole mass analyzers, ion traps such as quadrupole ion trap, quadrupole ion trap, orbitrap, quadrupole ion trap, Fourier transform ion cyclotron resonance mass spectrometry (FTMS), Ion cyclotron resonance (ICR) or combinations of the above. In the methods of the invention, the direct mass spectral analysis is carried out by MALDI - time of flight (MALDI-TOF) mass spectrometry.

Also disclosed is a method of providing a prognosis for a pregnancy comprising:

a) obtaining a sample
b) subjecting the sample to direct mass spectral analysis; and
c) comparing the spectra resulting from said analysis to mass spectral spectra obtained from a sample from a normal healthy ongoing pregnancy to determine whether said spectra from said sample is indicative of a poor prognosis for pregnancy.

Preferably the disclosed method provides a prognosis and/or detection of a disorder of pregnancy, and the spectra resulting from the analysis are compared to those from a woman with a normal healthy pregnancy, in particular a normal healthy singleton pregnancy

Alternatively the disclosed method provides a prognosis of successful implantation of a cultured embryo, and the spectra resulting from the analysis are compared to those from the fluid surrounding an embryo which has successfully implanted i.e. resulted in a viable pregnancy.

In this specification, the verb "comprise" has its normal dictionary meaning, to denote non-exclusive inclusion. That is, use of the word "comprise" (or any of its derivatives) to include one feature or more, does not exclude the possibility of also including further features. The word "preferable" (or any of its derivatives) indicates one feature or more that is preferred but not essential.

The application will now be described in the examples below which refer to the following figures:

Figure 1 illustrates the regions of interest in the spectra generated from a sample. Figure 2 shows the MALDI-ToF mass spectrum obtained from the overlay of three spectra from the three samples analyzed in this example; normal, molar and choriocarcinoma pregnancy urine samples. The neat normal pregnancy urine shows a peak at 36,687 m/z represents the normal condition. The molar pregnancy urine, shows a peak at 38,405 m/z within a range of 37,600 m/z to 39,200 m/z and the choriocarcinoma urine sample shows a peak at 38,803 m/z which lies within the 37,900 m/z to 39,600 m/z range.

Figure 3 shows the MALDI-TOF mass spectra (overlay and then stacked) of expected peak regions in 14,000 m/z to 100,000 m/z range for normal pregnancy urine compared to those of hyperemesis gravidarum. The peaks indicated in the top spectra at 23897 m/z, 36123 m/z and 38405 m/z are indicative of the normal state. In the lower half of the figure peaks at 33670 m/z

## Claims

1. A method of providing a prognosis of successful implantation of a cultured embryo, comprising:

   subjecting a sample to direct mass spectral analysis, wherein the spectra obtained from the direct mass spectral analysis is normalised; and
   comparing the normalised spectra obtained with the spectra obtained from a sample of a successfully implanted embryo;
   wherein the mass spectral analysis is carried out using Matrix Assisted Laser Desorption Ionization Time of Flight mass spectrometry ("MALDI-ToF MS") and the sample consists of culture fluid surrounding the embryo in culture; and
   wherein the spectra obtained from the mass spectral analysis is examined for the presence of abnormal peaks in the ranges 22,000-26,000 m/z and 35,000-40,000 m/z indicative of an embryo which is less likely to implant successfully.

2. A method as claimed in claim 1, wherein the sample comprises a neat sample.

3. A method as claimed in claim 1, wherein the sample is diluted prior to direct mass spectral analysis.

4. A method as claimed in claim 3, wherein the sample is not processed prior to dilution.

5. A method as claimed in claim 4, wherein the sample is not processed by removal of contaminating proteins such as albumin.

6. A method as claimed in any preceding claim, wherein the sample is taken 3-5 days post-fertilisation.

7. A method as claimed in any preceding claim, wherein the sample is taken 0.5 to 12 hours before the embryo is to be transferred to the recipient.

8. A method as claimed in any preceding claim, wherein each sample is compared against a reference spectral model of expected mass between 500 m/z to 100,000 m/z determined from statistical analysis of a collection of samples obtained from normal ongoing pregnancies.

**Patentansprüche**

1. Verfahren zum Bereitstellen einer Prognose für eine erfolgreiche Implantation eines kultivierten Embryos, umfassend:

   Unterziehen einer Probe gegenüber direkter Massenspektralanalyse, wobei die aus der direkten Massenspektralanalyse erhaltenen Spektren normalisiert sind; und
   Vergleichen der normalisierten Spektren mit den aus einer Probe eines erfolgreich implantierten Embryos erhaltenen Spektren;
   wobei die Massenspektralanalyse unter Verwendung von matrixunterstützter Laserdesorptionsionisations-Flugzeitmassenspektrometrie (Matrix Assisted Laser Desorption Ionization Time of Flight mass spectrometry, MALDI-ToF-MS) durchgeführt wird und die Probe aus das Embryo in Kultur umgebendem Kulturfluid besteht; und
   wobei die aus der Massenspektralanalyse erhaltenen Spektren bezüglich des Vorliegens abnormaler Peaks in den Bereichen 22 000-26 000 m/z und 35 000-40 000 m/z, die für ein Embryo indikativ sind, das weniger wahrscheinlich erfolgreich implantiert wird, untersucht werden.

2. Verfahren nach Anspruch 1, wobei die Probe eine unverdünnte Probe umfasst.

3. Verfahren nach Anspruch 1, wobei die Probe vor der direkten Massenspektralanalyse verdünnt wird.

4. Verfahren nach Anspruch 3, wobei die Probe vor der Verdünnung nicht verarbeitet wird.

5. Verfahren nach Anspruch 4, wobei die Probe nicht durch Entfernen verunreinigender Proteine wie Albumin verarbeitet wird.

6. Verfahren nach einem der vorstehenden Ansprüche, wobei die Probe 3-5 Tage nach der Befruchtung entnommen wird.

7. Verfahren nach einem der vorstehenden Ansprüche, wobei die Probe 0,5 bis 12 Stunden vor Übertragung des Embryos in die Empfängerin entnommen wird.

8. Verfahren nach einem der vorstehenden Ansprüche, wobei jede Probe mit einem Referenzspektralmodell erwarteter Masse zwischen 500 m/z bis 100 000 m/z, bestimmt gemäß statistischer Analyse einer Sammlung von aus normalen laufenden Schwangerschaften erhaltenen Proben, verglichen wird.

**Revendications**

1. Procédé de fourniture d'un pronostic d'implantation réussie d'un embryon de culture, comprenant :

   la soumission d'un échantillon à une analyse spectrale de masse directe, dans lequel le spectre obtenu à partir de l'analyse spectrale de masse directe est normalisé ; et
   la comparaison du spectre normalisé obtenu au spectre obtenu à partir d'un échantillon d'un embryon implanté avec succès ;
   dans lequel l'analyse spectrale de masse est effectuée en utilisant une spectrométrie de masse à temps de vol par désorption-ionisation par impact laser assistée par matrice (« MALDI-ToF MS ») et l'échantillon est constitué de fluide de culture entourant l'embryon en culture ; et
   dans lequel le spectre obtenu à partir de l'analyse spectrale de masse est examiné pour déceler la présence de pics anormaux dans les plages 22 000 à 26 000 m/z et 35 000 à 40 000 m/z indicatifs d'un embryon qui est moins susceptible de s'implanter avec succès.

2. Procédé selon la revendication 1, dans lequel l'échantillon comprend un échantillon pur.

3. Procédé selon la revendication 1, dans lequel l'échantillon est dilué avant analyse spectrale de masse directe.

4. Procédé selon la revendication 3, dans lequel l'échantillon n'est pas traité avant dilution.

5. Procédé selon la revendication 4, dans lequel l'échantillon n'est pas traité par élimination de protéines contaminantes telles que l'albumine.

6. Procédé selon l'une quelconque des revendications précédentes, dans lequel l'échantillon est prélevé 3 à 5 jours après fécondation.

7. Procédé selon l'une quelconque des revendications précédentes, dans lequel l'échantillon est prélevé 0,5 à 12 heures avant que l'embryon doive être transféré au bénéficiaire.

8. Procédé selon l'une quelconque des revendications précédentes, dans lequel chaque échantillon est comparé vis-à-vis d'un modèle spectral de référence de masse attendue entre 500 m/z et 100 000 m/z déterminée à partir d'une analyse statistique d'une collection d'échantillons obtenus à partir de grossesses normales en cours.

Figure 1

Figure 2

(a)

(b)

Figure 3

Figure 4

EP 3 198 279 B1

Pregnancy Losses

Negative Pregnancy

Ongoing Pregnancy

min -[lower quartile – median – upper quartile]- max

Figure 5

Figure 6

Best Embryo Probability Score

EP 3 198 279 B1

Figure 7

An example of 5 HotSpots

EP 3 198 279 B1

Figure 8

## Identify True Positives

If Σ 15800-16100 Th >0.3
and
If Σ 36400 Th >3

=

| Outcome | Correctly Identified |
|---|---|
| Ongoing Pregnancies | 39/43 = **90%** |
| SAB | 2/13 = **15%** |
| Biochemical | 3/10 = **30%** |
| Aneuploid (PGS) | 2/15 = **13%** |
| Negative | 11/34 = **32%** |

42

**+** Remove Fetal Anomalies and Biochem

If $\frac{Σ\ 9000\text{-}9500\ Th}{Σ\ 8400\text{-}8700\ Th}$ ≥10

=

| Outcome | Correctly Identified |
|---|---|
| Ongoing Pregnancies | 27/43 = **62%** |
| SAB | 3/13 = **23%** |
| Biochemical | 6/10 = **60%** |
| Aneuploid (PGS) | 15/15 = **100%** |
| Negative | 20/34 = **59%** |

49%

**+** Remove high risk SAB and Negatives

If 28000 Th ≥0.03

=

| Outcome | Correctly Identified |
|---|---|
| Ongoing Pregnancies | 27/43 = **62%** |
| SAB | 8/13 = **62%** |
| Biochemical | 6/10 = **60%** |
| Aneuploid (PGS) | 15/15 = **100%** |
| Negative | 23/34 = **68%** |

57%

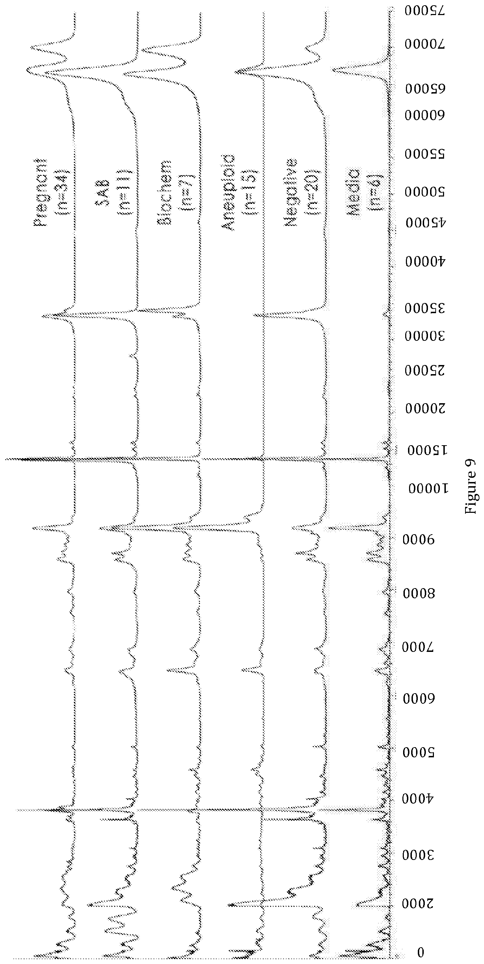

Figure 9

Figure 10

Figure 11

(a)

(b)

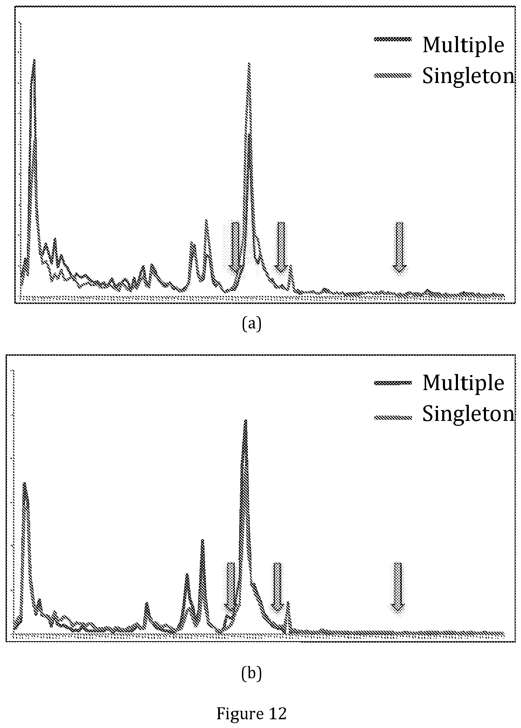

Figure 12

**Decision Tree**

HotSpot
12500-12550

> 0.004097    Single Embryo

≤ 0.004097

HotSpot
10300-10350

> 0.009914    Multiple Embryo

≤ 0.009914

HotSpot
9600-9650

> 1.422213    Multiple Embryo

≤ 1.422213

Single Embryo

12500 to 12550 <= 0.004097
|   10300 to 10350 <= 0.009914
|   |   9600 to 9650 <= 1.422213: single (9.0)
|   |   9600 to 9650 > 1.422213: multi (3.0/1.0)
|   10300 to 10350 > 0.009914: multi (3.0)
12500 to 12550 > 0.004097: single (102.0/1.0)

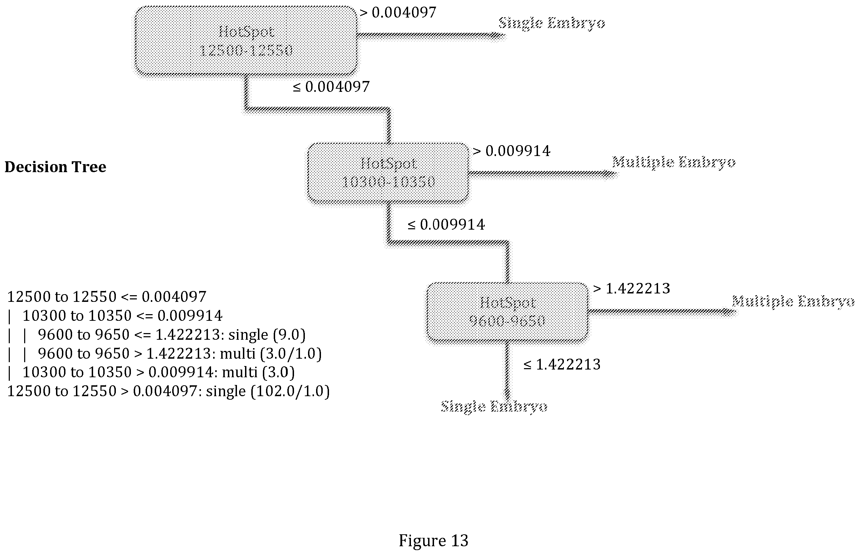

Figure 13

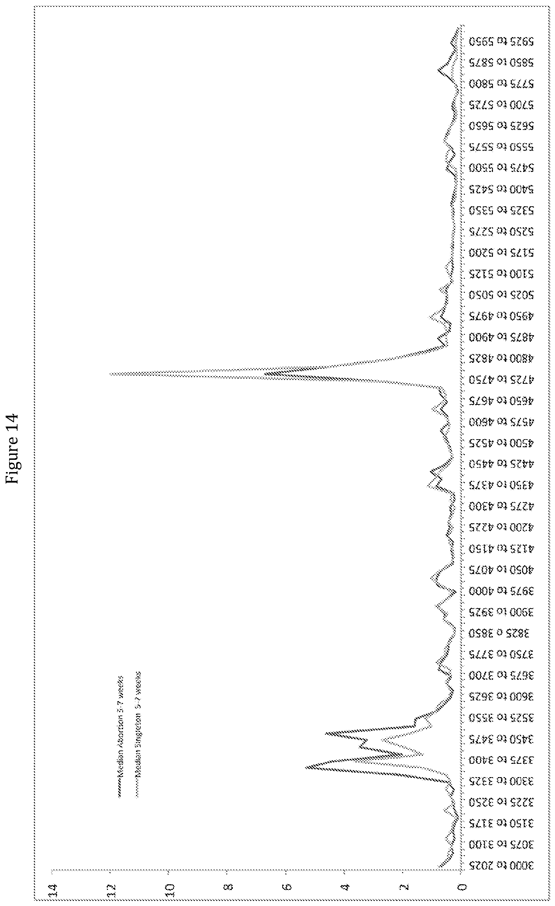

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009032288 A
- US 20100099135 A
- WO 2011075842 A
- WO 2012066057 A
- EP 2975402 A
- WO 2015173107 A
- US 20090075293 A

**Non-patent literature cited in the description**

- **NATASA KAHRIC-JANICIC et al.** *THYROID,* vol. 17 (4), 303-311